# EUROPEAN PATENT APPLICATION

(11) **EP 1 205 487 A1**
(43) Date of publication of application: **15.05.2002**
(21) Application number: 00123519.1
(22) Date of filing: 27.10.2000
(51) Int. Cl.: C07K 5/078, C07K 5/087, C07K 5/093

(54) **Bicyclic dipeptide isosteres, their preparation and use as beta-turn mimics**

(71) Applicant: Universita Degli Studi di Firenze, 50121 Florence (IT)
(72) Inventor: Cordero, Franca Maria, 50131 Firenze (IT); Brandi, Alberto, 50019 Sesto Fiorentino (FI) (IT); Valenza, Silvia, 50065 Pontassieve (FI) (IT); Machetti, Fabrizio, 59100 Prato (IT)
(74) Representative: Gervasi, Gemma, Dr.

(57) **Abstract**

The present invention relates to bicyclic dipeptide isosteres of general formula (I) their preparation by a cycloaddition/cyclization process and their use as Beta-turn Mimics.

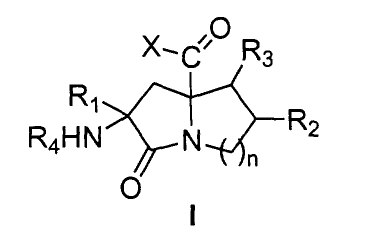

## Description

### Field of the invention

The present invention refers to bicyclic dipeptide isosteres, to a process for their preparation and to their use as Beta-turn Mimics.

### State of the art

Peptides play important roles in the regulation of many different biological processes. The determination of the receptor-bound conformation of a peptide, useful for the design of peptide mimetics, is a difficult task considering the high flexibility of these molecules.

Constrained analogues of peptides have been used to search the relationship between peptide conformation and activity and create more and more effective drugs.

Several constrained dipeptide surrogates have been designed to replace the central residues of beta-turn motifs (for a recent review see S. Hanessian, G. McNaughton-Smith, H.-G. Lombard, W. D. Lubell Tetrahedron 1997, 53, 12789-12854) because of the importance of these secondary structures in protein folding and recognition.

Of particular interest in this area are Xaa-Pro dipeptide analogues obtained by tethering the α-carbon of the *N*-terminal amino acid residue to the proline 2-position in order to control the cis-prolyl amide geometry (for two examples of a two atom tether see: D. Gramberg, C. Weber, R. Beeli, J. Inglis, C. Bruns, J. A. Robinson Helv. Chim. Acta 1995, 78,1588; K. Kim, J.-P. Dumas, J. P. Germanas J. Org. Chem. 1996, 61, 3138).

For an example of a three atom tether see: T. P. Curran, P. M. McEnaney, Tetrahedron Lett. 1995, 36, 191).

### Summary of the invention

The present invention refers to bicyclic dipeptide isosteres of following general formula (I), 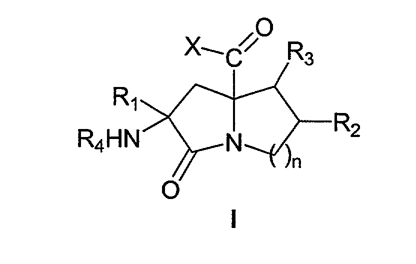 wherein:
n is an integer from 1 to 10
R₁, is chosen in the group consisting of H, C₁₋₁₆alkyl, aryl, an amino acid side-chain;
R₂, is chosen in the group consisting of H, C₁₋₁₆alkyl, OR^{I};
R₃, is chosen in the group consisting of H, C₁₋₁₆alkyl, OR^{I} or
R₂ and R₃ taken together are an aryl-fused group;
R₄, is chosen in the group consisting of H, C₁₋₁₆alkyl; a protecting group; -C(O)CH(amino acid side-chain)NR^{II}R^{III}, -C(O)(1-R^{II}-pyrrolidin-2-yl); -C(O)(1-R^{II}-piperidin-2-yl); -C(O)CH₂CH₂NR^{II}R^{III};
X is chosen in the group consisting of OR^{I}, NR^{IV}R^{V};
R^{I} is chosen in the group consisting of H, C₁₋₁₆alkyl, a protecting group;
R^{II} and R^{III}, same or different, are chosen in the group consisting of: H, C₁₋₁₆alkyl, a protecting group, -C(O)CH(amino acid side-chain)NR^{II}R^{III}, -C(O)(1-R^{II}-pyrrolidin-2-yl); -C(O)(1-R^{II}-piperidin-2-yl); -C(O)CH₂CH₂NR^{II}R^{III};
R^{IV} and R^{V}, same or different, are chosen in the group consisting of: H, C₁₋₁₆alkyl, a protecting group, -CH(amino acid side-chain)COX, -CH₂CH₂C(O)X or
R^{IV} and R^{V} taken together are: -CH(COX)CH₂CH₂CH₂-, -CH(COX)CH₂CH₂CH₂CH₂-;
the above said alkyl- and aryl-groups, being, possibly, substituted.

The application refers also to a process for the preparation of the above said compounds and their use for inducing beta-turn when incorporated into a peptide.

### Detailed description of the invention

The bicyclic lactams of formula (I) according to the invention allow to overcome the above mentioned problems. In fact the hereinafter described compounds incorporate a carboxyl and an amino group in geometrically suitable position and orientation for peptide coupling and beta-turn mimicking. Moreover the present compounds can be easily synthesised in few steps starting from cyclic 2-carboxyl-nitrones (II) and α,β-unsatured acid derivatives (III). The easy introduction of different substituents on the basic bicyclic ring system allows to modulate the hydrophobic nature of the system.

Therefore the present compounds make possible a convenient approach to Xaa-Pro (I, n=1, R₂=R₃=H) dipeptide surrogate in which the α-carbon of Xaa is tethered to the proline 2-carbon through a -CH₂- bridge furnishing a lower flexibility than other dipeptide analogues described in literature which have a longer tether to bridge the two amino acid residues.

Another point of interest is represented by the fact that it is possible to choose various substituent R₁ by simply changing the dipolarophile (III). The introduction of different side chains provides a very useful opportunity to modulate the polarity, the conformation and the activity of the final peptide.

Finally, the method is easily applicable to combinatorial synthesis of constrained Xaa-Pro analogues of structure (I).

As already said the invention refers to a new an advantageous process for the preparation of the above defined compounds of formula (I).

According to the present invention in the compounds of formula (I) as above defined:
protecting group means any group capable of preventing the atom to which it is attached from participating in an undesired reaction or bonding, as commonly used in synthesis reactions.

Preferred protecting groups are those which prevent reaction or bonding of oxygen, nitrogen, carboxylic acids, thiols, alcohols, amines and the like. Such groups and their preparation and introduction are conventional in the art and include, for example, for COOH group: methyl, ethyl, tert-butyl, benzyl, allyl esters; for the NH group: Boc, Fmoc, Cbz, Bn, Bz; for the OH group: benzyl, tert-butyl; acetals, esters, trialkylsilylethers.

Amino acid side-chain means the different amino acid side-chains moieties of any one of the twenty L or D natural α-amino acids (i.e. : -H of glycine (Gly); -CH₃ of alanine (Ala); -CH(CH₃)₂ of valine (Val); -CH₂CH(CH₃)₂ of leucine (Leu); -CH(CH₃)CH₂CH₃ of isoleucine (IIe); -CH₂OH of serine (Ser); -CH(OH)CH₃ of threonine (Thr); -CH₂SH of cysteine (Cys); -CH₂CH₂SCH₃ of methionine (Met); -CH₂-(phenyl) of phenylalanine (Phe); -CH₂-(phenyl)-OH of tyrosine (Tyr); -CH₂-(1H-indol-3-yl) of tryptophan (Trp); -CH₂COOH of aspartic acid (Asp); -CH₂C(O)NH₂ of asparagine (Asn); -CH₂CH₂COOH of glutamic acid (Glu); -CH₂CH₂C(O)NH₂ of glutamine (Gln); -CH₂CH₂CH₂N(H)C(NH₂)NH of arginine (Arg); -CH₂-(1H-imidazol-4-yl) of histidine (His); -CH₂(CH₂)₃NH₂ of lysine (Lys)) or also of non naturally or non proteinogenic occurring amino acids like: -CH₂(CH₂)₂CH₃ of norleucine (Nle), -CH₂CH₂CH₃ of norvaline (NVa), -phenyl of α-phenyl glycine, or others well known amino acids in the peptide art. The above specified amino acid side chains can obviously bear the protecting groups (Pg) normally used in the art for protecting purposes.

In the compounds of formula (I), as above defined groups C₁₋₁₆alkyl represent linear or branched alkyl radicals as for example: methyl, ethyl, propyl, isopropyl, butyl, pentyl, hexyl, heptyl, octyl and so on up to hexadecyl and their corresponding isomers.

According to the invention the term aryl means preferably: phenyl, naphthyl, pyridyl, and quinolinyl groups.

When R₂ and R₃ taken together are an aryl-fused group this is preferably: phenyl, naphthyl, pyridyl, and quinolinyl groups.

The above specified aryl groups can be substituted with one or more, preferably one or two, moieties chosen from the groups consisting of: C₁₋₁₆alkyl, X, NR^{II}R^{III}, -C(O)C₁₋₁₆alkyl, -C(O)X, -CH₂X, -CH₂NR^{II}R^{III}, halogen, cyano, nitro.

Wherein X, R^{I}, R^{II} and R^{III} are as above defined and the term halogen represent fluorine, chlorine, bromine, iodine.

The synthetic process according to the invention involves only two steps to assemble the bicyclic scaffold of formula (IV) starting from a cyclic nitrone of formula (II) and a α,β-unsatured acid derivative of formula (III). The defined compounds of general formula (I) is then obtained from (IV) through few common functional group interconversions.

According to the invention the compounds of formula (I) can be prepared starting from compounds of general formula (II) and (III) 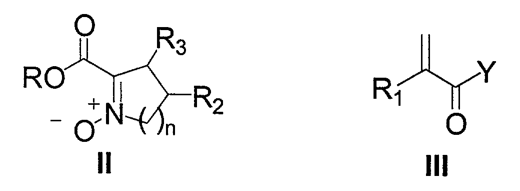 wherein:
n, R₁, R₂ and R₃ are as above defined;
R is H, or a suitable protecting group as above defined, or a chiral auxiliary;
Y is chosen in the group consisting of OR^{VI}, NR^{VII}R^{VIII};
R^{VI}, is chosen in the group consisting of H, C₁₋₄alkyl, a protecting group, a chiral auxiliary;
R^{VII} and R^{VIII}; same or different, are chosen in the group consisting of: H, C₁₋₄alkyl, a protecting group, a chiral auxiliary.

Non-limiting examples of chiral auxiliaries include enantiomerically pure menthyl, 2-phenylcyclohexyl, phenylmenthyl, naphthylmenthyl, etc.

The cyclic nitrones (II) can be prepared for example starting from the corresponding cyclic amines (V) or hydroxylamines (VI) (wherein n, R₂ and R₃ are as above defined) as shown in the Scheme 1 according to known procedures.

On the other hand, the parent cyclic nitrones (II), wherein n = 1 - 10 and R₂ = R₃ = H, can be directly obtained by oxidation of proline esters, pipecolic acid esters, azepane-, azocane-, azonane-, azecane-, azacycloundecane-, azacyclododecane-, azacyclotridecane- or azacyclotetradecane-2-carboxylic esters respectively.

The acid derivatives (III) are commercial available or can be prepared according to known procedures.

As it can be seen from the Scheme 2 the preparation of the compounds (I) according to the invention involves, in the Step 1, the 1,3-dipolar cycloaddition of the nitrone (II) and the dipolarophile (III) to give the isoxazolidine derivative (X).

Because Step I is unaffected by the solvent it can be run in any kind of solvent.

Preferably the reaction is performed in an environment friendly solvent as water, at a temperature comprised between 25°C and 100°C, preferably at 60°C, for a time comprised between 1 and 24 hours.

The Step 2, is a domino reaction in which the reductive opening of the isoxazolidine (X) is followed by an intramolecular cyclization to give the bicyclic lactam intermediate (IV). Reduction of the N-O bond can be achieved, for example, by Zn in acetic acid, hydrogenolysis on Pd, Pt or Ni Raney, Mo(CO)₆/H₂O, Fe(CO)₅, Sml₂, etc. The best results were obtained by hydrogenation using Pd(OH)₂ on carbon as catalyst and performing the reaction in methanol, at 1 atm, and room temperature for a time comprised between 1 and 12 hours, preferably in the presence of 10 equivalents of CH₃CO₂H.

At this stage, if necessary, the initial OR group can be substituted with a different X moiety, to obtain a final dipeptide surrogate more suitable for the peptide synthesis in solution or in solid phase. For example a methyl ester can be transformed in allylic or benzylic ester through a trans-esterification reaction preferably using the alcohol as solvent and in the presence of 1,8-diazabicyclo[5.4.0]undec-7-ene (DBU) and LiBr, at room temperature for 1 hour.

It is also possible to resolve the racemic IV to obtain enantiomerically pure alcohols IV which will lead to enantiomerically pure I. For example, IV can be esterified to IV' wherein R₅ is an enantiomerically pure acid residue (preferably Mosher acid or camphorsulfonic acid). The diastereomeric esters IV^{I} can be separated, by crystallization or by chromatography on silica gel, and the hydroxy function restored by basic hydrolysis or transesterification.

The subsequent reaction concerns the conversion of the hydroxy function in amine to produce (I) wherein R₄ = H, through any conventional method. The reaction is preferably performed through the formation of the corresponding azide of formula XI, 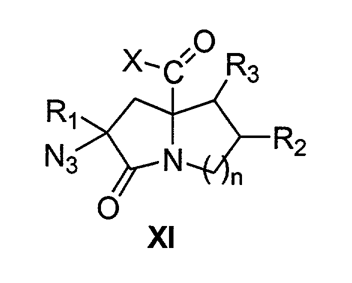 wherein:
n, R_{1,},R₂,R₃ and X are as above defined, for example using the system diphenylphosphoryl azide (dppa)/DBU or dppa/4-(dimethylamino)pyridine (DMAP), or reacting the alcohol (IV) first with methanesulfonyl chloride (MsCI) and then with sodium azide. Then the azido group can be easily reduced to amine preferably by Ni Raney, PPh₃/H₂O or hydrogenolysis over Lindlar catalyst.

The free amine group of the bicyclic lactam (I, R₄ = H) can be protected with a suitable protecting group or directly coupled with the carboxyl group of any amino acid.

The invention will be better understood in the light of the following Examples.

### EXAMPLE 1

### Preparation of (2R*,3aR*)-tetrahydro-2-(aminocarbonyl)-pyrrolo[1,2-b]isoxazole-3a(4H)-carboxylic acid methyl ester and (2S*,3aR*)-tetrahydro-2-(aminocarbonyl)-pyrrolo[1,2-b]isoxazole-3a(4H)-carboxylic acid methyl ester [compounds (2R*,3aR*)-X and (2S*,3aR*)-X wherein R = Me, R₁ = R₂ = R₃ = H, Y = NH₂, n = 1, endo and exo adducts]

A solution of II (wherein R = Me, R₂ = R₃ = H, n = 1) (1.1 g, 7.69 mmol) and III (wherein , R₁ = H, Y = NH₂) (1.09 g, 15.4 mmol) in bidistilled H₂O (2.2 mL) was stirred at 60 °C for 14 hrs. The mixture was thoroughly extracted with CH₂Cl₂ and the organic extracts were dried with Na₂SO₄, filtered and concentrated. The crude product was crystallised from AcOEt yielding (2*S**,3a*R**)-X (wherein R = Me, R₁ = R₂ = R₃ = H, Y = NH₂, n = 1, exo adduct) as a white cristalline solid (0.71g, 23%). The mother liquor was concentrated and purified by column chromatography on silica gel (eluent: initially CHCl₃ : *i*PrOH : conc. NH₄OH, 1000 : 10 : 0.1 then the polarity was gradually increased) to obtain other (2*S**,3a*R**)-X (wherein, R = Me, R₁ = R₂ = R₃ = H, Y = NH₂, n = 1, *exo* adduct) as a white cristalline solid (0.28g, 17%) and its diastereoisomer (2*R**,3a*R**)-X (wherein R = Me, R₁ = R₂ = R₃ = H, Y = NH₂, n = 1, *endo* adduct) (0.49 g, 37%) as a yellow viscous oil.

(2*S**,3a*R**)-X (wherein R = Me, R₁ = R₂ = R₃ = H, Y = NH₂, n = 1, *exo* adduct): R_{f} = 0.20 (CHCl₃ : *i*PrOH : conc. NH₄OH, 1000 : 10 : 0.1); m.p. = 163-164 °C; ¹H NMR (200 MHz, CDCl₃) δ 6.87 (br s, 1H), 5.37 (br s, 1H), 4.59 (dd, *J* = 8.8, 4.8 Hz, 1H), 3.76 (s, 3H), 3.44-3.27 (m, 2H), 3.18 (dd, *J* = 13.2, 4.8 Hz, 1H), 2.63 (dd, *J* = 13.2, 8.8 Hz, 1H), 2.40-2.22 (m, 1H), 2.17-2.09 (m, 3H).

(2*R**,3a*R**)-X (wherein R = Me, R₁ = R₂ = R₃ = H, Y = NH₂, n = 1, *endo* adduct): R_{f} = 0.13 (CHCl₃ : *i*PrOH : conc. NH₄OH, 1000 : 10 : 0.1); ¹H NMR (200 MHz, CDCl₃) δ 6.43 (br s, 1 H), 6.35 (br s, 1 H), 4.50 (dd, *J* = 9.2, 7.5 Hz, 1H), 3.73 (s, 3H), 3.35-3.08 (m, 2H), 3.20 (dd, *J* = 12.8, 7.5 Hz, 1H), 2.31-2.20 (m, 1H), 2.25 (dd, *J* = 12.8, 9.1 Hz, 1H), 2.08-1.84 (m, 3H).

### EXAMPLE 2

### Preparation of (2S*,7aR*)-tetrahydro-2-hydroxy-3-oxo-1H-pyrrolizine-7a(5H)-carboxylic acid methyl ester [compound (2S*,7aR*)-IV wherein R = Me, R₁ = R₂ = R₃ = H, n =1]

A mixture of (2*S**,3a*R**)-X (wherein R = Me, R₁ = R₂ = R₃ = H, Y = NH₂, n = 1; prepared according to the example 1) (0.68 g, 3.17 mmol), AcOH (2 mL, 31.7 mmol) and in MeOH (26 mL) was hydrogenated over 20% Pd(OH)₂ (80 mg) at room temperature and atmospheric pressure for 12 hrs. The catalyst was removed by filtration on a short pad of Celite. The filtrate was concentrated, diluted with CH₂Cl₂ (50 mL) and treated with K₂CO₃ and Na₂SO₄ for 30 min. The mixture was filtered and concentrated to give analytically pure (2*S**,7a*R**)-IV (wherein R = Me, R₁ = R₂ = R₃ = H, n =1) (0.62 g, 98%) as a white solid.

(2*S**,7a*R**)-IV (wherein R = Me, R₁ = R₂ = R₃ = H, n =1): m.p. = 105-106 °C; ¹H-NMR (500 MHz, CDCl₃) δ 4.71 (dd, *J* = 10.5, 7.5 Hz, 1H), 4.36 (br s, 1H), 3.74 (s, 3H), 3.68 (dt, *J* = 11.4, 7.8 Hz, 1H), 3.16 (m, 1H), 3.00 (dd, *J* = 12.7, 7.5 Hz, 1H), 2.46 (ddd, *J* = 12.7, 7.5, 3.2 Hz, 1H), 2.12-1.99 (m, 2H), 1.96 (dd, *J* = 12.7, 10.5 Hz, 1H), 1.73 (dt, *J* = 12.7, 9.7 Hz, 1H).

### EXAMPLE 3

### Preparation of (2R*,7aR*)-tetrahydro-2-hydroxy-3-oxo-1H-pyrrolizine-7a(5H) carboxylic acid methyl ester [compound (2R*,7aR*)-IV wherein R = Me, R₁ = R₂ = R₃ = H, n =1]

Following the procedure described in the example 2, (2*R**,3a*R**)-X (wherein R = Me, R₁ = R₂ = R₃ = H, Y = NH₂, n = 1; prepared according the example 1) gave analytically pure (2*R**,7a*R**)-IV (wherein R = Me, R₁ = R₂ = R₃ = H, n =1) (98% yield) as a white solid.

(2*R**,7a*R**)-IV (wherein R = Me, R₁ = R₂ = R₃ = H, n =1): m.p. = 130-131 °C; ¹H-NMR (200 MHz, CDCl₃) δ 4.38 (dd, *J* = 7.0, 1.5 Hz, 1H), 4.49 (br s, 1H), 3.75 (s, 3H), 3.59 (dt, *J* = 11.7, 8.1 Hz, 1H), 3.28 (ddd, *J* = 11.7, 8.4, 4.8 Hz, 1H), 2.53 (dd, *J* = 14.3, 1.5 Hz, 1H), 2.34 (ddd, *J* = 9.5, 6.6, 2.9 Hz, 1H), 2.25 (dd, *J* = 14.3, 7.0 Hz, 1H), 2.18-1.38 (m, 2H), 1.60 (dt, *J* = 12.4, 9.9 Hz, 1H).

### EXAMPLE 4

### Isomerization of (2R*,7aR*)-tetrahydro-2-hydroxy-3-oxo-1H-pyrrolizine-7a(5H)-carboxylic acid methyl ester [compound (2R*,7aR*)-IV wherein R = Me, R₁ = R₂ = R₃ = H, n =1] to (2S*,7aR*)-tetrahydro-2-hydroxy-3-oxo-1H-pyrrolizine-7a(5H)-carboxylic acid methyl ester [compound (2S*,7aR*)-IV wherein R = Me, R₁ = R₂ = R₃ = H, n =1]

A solution of compound (2*R**,7a*R**)-IV (wherein, R = Me, R₁ = R₂ = R₃ = H, n =1; prepared according to the example 3) (0.08 g, 0.4 mmol) in MeOH (0.6 mL) was treated with 1N NaOH at 0 °C and the mixture was heated to 60 °C for 6 hrs. The solution was neutralised by treatment with Dowex 50 (H⁺ form), filtered and treated with an ethereal solution of CH₂N₂. After 12 hrs the solvents were removed under reduced pressure to give analytically pure product (2*S**,7a*R**)-IV (wherein R = Me, R₁ = R₂ = R₃ = H, n =1) (0.036 g, 45%) as a white solid.

### EXAMPLE 5

### Preparation of (2S*,7aR*)-tetrahydro-2-hydroxy-3-oxo-1H-pyrrolizine-7a(5H)-carboxylic acid allyl ester [compound (2S*,7aR*)-IV wherein, R = All, R₁ = R₂ = R₃ = H, n = 1].

Methyl ester (2*S**,7a*R**)-IV (wherein R = Me, R₁ = R₂ = R₃ = H, n =1; prepared according to the example 2 or to the example 4) (0.59 g, 2.996 mmol) and LiBr (1.285 g, 14.8 mmol) were dissolved in absolute allyl alcohol under dry N₂. Freshly distilled DBU (221 µL, 1.48 mmol) was added and the solution was stirred at room temperature for 1 h. The mixture was concentrated, neutralised with 1N HCI and extracted with AcOEt . The organic phase was washed with brine, dried with Na₂SO₄ and concentrated. The crude product (0.614 g) was purified by column chromatography on silica gel (eluent: CH₂Cl₂ : MeOH, 20 :1, *R*_{*f*} = 0.23) to afford analitically pure (2*S**,7a*R**)-IV (wherein R = All, R₁ = R₂ = R₃ = H, n =1) (0.47 g, 70%) as a white solid.

(2*S**,7a*R**)-IV (wherein R = All, R₁ = R₂ = R₃ = H, n =1): *R*_{*f*} = 0.23 (CH₂Cl₂: MeOH, 20 :1); ¹H-NMR (200 MHz, CDCl₃) δ 5.90 (ddd, *J* = 17.2, 10.4, 5.8 Hz, 1H), 5.38-5.24 (m, 2H), 4.73 (dd, *J* = 9.2, 8.8 Hz, 1H), 4.64 (dt, *J* = 5.8, 1.4 Hz, 2H), 4.26 (br s, 1H), 3.71 (dt, *J* = 11.5, 8.0 Hz, 1H), 3.18 (dddd, *J* = 12.4, 8.0, 5.0, 1.4 Hz, 1H), 3.03 (dd, *J* = 13.0, 7.8 Hz, 1H), 2.50 (ddd, *J* = 12.4, 6.4, 4.0 Hz, 1H), 2.15-2.02 (m, 2H), 1.98 (dd, *J* = 13.0 10.4 Hz, 1H), 1.74 (dt, *J* = 12.4, 9.6 Hz, 1H).

### EXAMPLE 6

### Preparation of (2S*,7aR*)-tetrahydro-2-[(methylsulfonyl)oxy]-3-oxo-1H-pyrrolizine-7a(5H)-carboxylic acid allyl ester [compound (2S*,7aR*)-IV^{I} wherein X = OAII, R₁ = R₂ = R₃ = H, R₅ = Ms, n =1].

TEA (1.88 mL, 13.5 mmol) and MsCI (0.418 mL, 5.4 mmol) were added with cooling in an ice/water bath to a solution of compound (2*S**,7a*R**)-IV (wherein R = All, R₁ = R₂ = R₃ = H, n =1; prepared according to the example 5) (0.777 g, 3.45 mmol) in CH₂Cl₂ (4 mL). The mixture was stirred in dry atmosphere at room temperature overnight and the resulting suspension was diluted with CH₂Cl₂ (100 mL), washed sequentially with H₂O and brine, dried with Na₂SO₄ and concentrated. The crude product (0.772 g) was purified by column chromatography on silica gel (eluent: AcOEt : petroleum ether, 1 :1) to afford analytically pure (2*S**,7a*R**)-IV^{I} (wherein X = OAII, R₁ = R₂ = R₃ = H, R₅ = Ms, n =1) (0.617 g, 60%) as a yellow oil.

(2*S**,7a*R**)-IV^{I} (wherein X = OAII, R₁ = R₂ = R₃ = H, R₅ = Ms, n =1): *R*_{*f*} = 0.26 ( AcOEt : petroleum ether, 1 :1); ¹H-NMR (200 MHz, CDCl₃) δ 5.91 (ddt, *J* = 17.2, 10.4, 5.8 Hz, 1H), 5.57 (dd, *J* = 9.4, 8.4 Hz, 1H), 5.39-5.28 (m, 2H), 4.66 (d, *J* = 5.8 Hz, 2H), 3.73 (dt, *J* = 11.8, 7.8 Hz, 1H), 3.30 (s, 3H), 3.29-3.10 (m, 1H), 3.15 (dd, *J* = 13.2, 8.0 Hz, 1H), 2.57 (ddd, *J* = 12.8, 6.6, 4.0 Hz, 1H), 2.22 (dd, *J* = 13.2, 10.2 Hz, 1H), 2.12-2.04 (m, 2H), 1.74 (dt, *J=* 12.8, 9.4 Hz, 1H).

### EXAMPLE 7

### Preparation of (2R*,7aR*)-2-azido-tetrahydro-3-oxo-1H-pyrrolizine-7a(5H)-carboxylic acid allyl ester [compound (2R*,7aR*)-XI wherein X = OAII, R₁ = R₂ = R₃ = H, n =1].

A solution of (2*S**,7a*R**)-IV^{I} (wherein X = OAII, R₁ = R₂ = R₃ = H, R₅ = Ms, n =1; prepared according to the example 6) (0.611 g, 2.0 mmol) and NaN₃ (0.261 g, 4.0 mmol) in DMF (6 mL) was heated to the reflux temperature for 1.5 h. The reaction mixture was diluted with CH₂Cl₂ (20 mL), treated dropwise with 1N HCI (5 mL) and vigorously stirred for 15 min. The separated organic phase was washed sequentially with H₂O and brine, dried with Na₂SO₄ and concentrated. The crude product (0.317 g) was purified by column chromatography on silica gel (eluent:

AcOEt: petroleum ether, 1 :1) to afford (2*R**,7a*R**)-XI (wherein X = OAII, R₁ = R₂ = R₃ = H, n =1) (0.269 g, 54%) as a yellow oil.

(2*R**,7a*R**)-XI (wherein X = OAII, R₁ = R₂ = R₃ = H, n =1):*R*_{*f*} = 0.22 (AcOEt : petroleum ether, 1 :1); ¹H-NMR (200 MHz, CDCl₃) δ 5.92 (ddt, J = 17.2, 10.4, 5.8

Hz, 1H), 5.35 (dq, *J*= 17.2, 1.0Hz, 1H), 5.29 (dq, *J* = 10.4, 1.0 Hz, 1H), 4.67 (dt, *J* = 5.8, 1.0 Hz, 2H), 4.21 (dd, *J* = 7.6, 1.4 Hz, 1H), 3.68 (dt, *J* = 11.8, 7.6 Hz, 1H), 3.33 (ddd, *J* = 11.8, 7.8, 5.4 Hz, 1H), 2.57 (dd, *J* = 14.2, 1.4 Hz, 1H), 2.34 (ddd, *J* = 12.2, 6.4, 3.8 Hz, 1H), 2.35 (dd, *J* = 14.2, 7.6 Hz, 1H), 2.20-2.05 (m, 2H), 1.69 (dt, *J* =12.4, 9.6 Hz, 1H).

### EXAMPLE 8

### Preparation of (2R*,7aR*)-2-amino-tetrahydro-3-oxo-1H-pyrrolizine-7a(5H)-carboxylic acid allyl ester [compound (2R*,7aR*)-I wherein X = OAII, R₁ = R₂ = R₃ = R₄ = H, n =1].

Compound (2*R**,7a*R**)-XI (wherein X = OAII, R₁ = R₂ = R₃ = H, n =1; according to the example 7) (0.036 g, 0.14 mmol) and PPh₃ (0.038 g, 0.14 mmol) were dissolved in dry THF (2 mL) and allowed to stand at room temperature for 1 h. Then H₂O (4 µL) was added and the mixture was stirred overnight at room temperature and concentrated. The crude product was purified by column chromatography on silica gel (eluent: initially CHCl₃: MeOH, 50 :1 then the polarity was gradually increased) to afford pure compound (2*R**,7a*R**)-I (wherein X = OAII, R₁ = R₂ = R₃ = R₄ = H, n =1) (0.013 g, 35 %) as a colourless oil.

(2*R**,7a*R**)-I (wherein X = OAII, R₁ = R₂ = R₃ = R₄ = H, n =1): *R*_{*f*} = 0.20 (CHCl₃: MeOH , 50 :1); ¹H-NMR (200 MHz, CDCl₃) δ 5.91 (ddt, *J* = 16.8, 10.6, 6.0 Hz, 1H), 5.39-5.25 (m, 2H), 4.66 (d, *J* = 6.0 Hz, 2H), 3.71-3.57 (m, 2H), 3.21 (ddd, *J* = 11.8, 9.2, 4.0 Hz, 1H), 2.51-2.24 (m, 3H), 2.16-1.94 (m, 2H), 1.86 (br s, 2H), 1.07 (dt, *J* = 12.2, 9.8 Hz, 1H).

### EXAMPLE 9

### Preparation of (2S*,7aR*)-2-azido-tetrahydro-3-oxo-1H-pyrrolizine-7a(5H)-carboxylic acid methyl ester [compound (2S*,7aR*)-XI wherein X = OMe R₁ = R₂ = R₃ = H, n = 1].

To a solution of compound (2*R**,7a*R**)-IV (wherein R = Me, R₁ = R₂ = R₃ = H, n =1; prepared according the example 4) (61 mg, 0.3 mmol) and DMAP (50 mg, 0.4 mmol) in toluene (0.6 mL) was added dppa (86 µL, 0.4 mmol). The mixture was heated at 65 °C for 3.5 h then purified by column chromatography on silica gel (eluent: initially petroleum ether then AcOEt : petroleum ether, 1 : 1) to afford analitically pure (2*S**,7a*R**)-XI (wherein X = OMe, R₁ = R₂ = R₃ = H, n =1) (56 mg, 81 %) as a colourless oil.

(2*S**,7a*R**)-XI (wherein X = OMe, R₁ = R₂ = R₃ = H, n =1): *R*_{*f*} = 0.44 (AcOEt : petroleum ether, 1:1); ¹H-NMR (200 MHz, CDCl₃) δ 5.10 (dd, *J* = 11.0, 7.7 Hz, 1H), 4.31 (s, 3H), 4.25 (dt, *J* = 12.0, 7.7 Hz, 1H), 3.79-3.66 (m, 1H), 3.49 (dd, *J* = 12.9, 7.7 Hz, 1H), 3.02 (ddd, *J* = 12.5, 6.6, 4.4 Hz, 1H), 2.71-2.55 (m, 2H), 2.39 (dd, *J* = 12.9, 11.0 Hz, 1H), 2.23 (dt, *J* = 12.5, 9.1 Hz, 1H).

### EXAMPLE 10

### Preparation of (2S*,7aR*)-2-amino-tetrahydro-3-oxo-1H-pyrrolizine-7a(5H)-carboxylic acid methyl ester [compound (2S*,7aR*)-I wherein X = OMe, R₁= R₂ = R₃ = R₄ = H, n =1].

To a solution of (2*S**,7a*R**)-XI (wherein X = OMe, R₁ = R₂ = R₃ = H, n =1; prepared according to the example 9) (0.030 g, 0.135 mmol) in MeOH (2 mL) was added dropwise a water suspension (1 mL) of Ni-Raney. The mixture was stirred at room temperature for 0.5 h, filtered through a short pad of Celite and concentrated under reduced pressure to give (2*S**,7a*R**)-I (wherein X = OMe, R₁ = R₂ = R₃ = R₄ = H, n =1) (0.022 g, 82 %) as a colourless oil.

(2*S**,7a*R**)-I (wherein X = OMe, R₁ = R₂ = R₃ = R₄ = H, n =1): *R*_{*f*} = 0.06 (AcOEt: petroleum ether =6 :1); ¹H-NMR (200 MHz, CDCl₃) δ 4.55 (br s, 2H), 3.92 (dd, *J* = 11.4, 7.8 Hz, 1H), 3.73 (s, 3H), 3.65 (dt, *J* = 11.4, 7.6 Hz, 1H), 3.22-3.09 (m, 1H), 2.96 (dd, *J*= 12.8, 7.6 Hz, 1H), 2.43 (ddd, *J*= 12.6, 6.4, 4.4 Hz, 1H), 2.11-1.96 (m, 2H), 1.76-1.70 (m, 1H), 1.63 (dd, *J* = 12.8, 11.6 Hz, 1H).

### EXAMPLE 11

### Preparation of (2R*,7aR*)-2-[[(2S)-2-[(1,1-dimethylethoxy)carbonyl]-2-[[(9H-fluoren-9-ylmethoxy)carbonyl]amino]-1-oxopropyl]amino]-tetrahydro-3-oxo-1H-pyrrolizine-7a(5H)-carboxylic acid allyl ester [compound (2R*,7aR*)-I wherein, X = OAII, R₁ = R₂ = R₃ = H, R₄ = C(O)CH(CH₂COOtBu)NHFmoc, n =11.

DIPEA (17.4 µL, 0.1 mmol) was added to a solution of compound (2*R**,7a*R**)-I (wherein X = OAII, R₁ = R₂ = R₃ = R₄ = H, n =1; prepared according to the example 8) (0.011 g, 0.05 mmol), L-Fmoc-NH-Asp(4-*t*Bu)-OH (0.026 g, 0.05 mmol) and PyBroP (0.023 g, 0.05 mmol) in CH₂Cl₂ (1 mL) with cooling in an ice/water bath. The mixture was allowed to stand at room temperature overnight, then was diluted with AcOEt (20 mL), filtered and washed sequentially with 5% KHSO₄, 5% NaHCO₃ and brine. The organic solution was dried over Na₂SO₄, filtered and concentrated. The crude product (0.034 g) was purified by column chromatography on silica gel (eluent: CHCl₃ : *i*PrOH, 100 : 1) to afford an equimolecular mixture of the two diastereoisomers (2*R**,7a*R**)-I (wherein X = OMe, R₁ = R₂ = R₃ = H, R₄ = C(O)CH(CH₂COOtBu)NHFmoc, n =1) (0.013 g, 43 %) as a colourless oil.

(2*R**,7a*R**)-I (wherein X = OMe, R₁ = R₂ = R₃ = H, R₄ = C(O)CH(CH₂COOtBu)NHFmoc, n =1, diastereomeric mixture): *R*_{*f*} = 0.15 (CHCl₃ : *i*PrOH, 100 : 1); HPLC: Rₜ = 28.2 min (from 20 to 80% of B in 30 min with 10 % of C; A = H₂O, B = CH₃CN, C = 10% TFA in H₂O, Phenomenex Luna C18 5 µ 250 x 4.6 mm); ¹H-NMR (200 MHz, CDCl₃) δ 7.77-7.74 (m, 4H), 7.62-7.59 (m, 4H), 7.46-7.18 (m, 10H), 6.01-5.77 (m, 3H), 5.34-5.21 (m, 3H), 4.69-4.39 (m, 14H), 4.27-4.20 (m, 2H), 3.73-3.59 (m, 2H), 3.29-3.18 (m, 2H), 3.00-2.81 (m, 2H), 2.71-2.03 (m, 10H), 1.76-1.56 (m, 4H), 1.44 (s, 18H).

### EXAMPLE 12

### Preparation of (2S*,7aR*)-2-[[(2S)-2-[[(1,1-dimethylethoxy)carbonyl]amino]-1-oxo-3-phenylpropyl]amino]-tetrahydro-3-oxo-1H-pyrrolizine-7a(5H)-carboxylic acid methyl ester [compound (2S*,7aR*)-I wherein X = OMe, R₁ = R₂ = R₃ = H, R₄ = C(O)CH(CH₂Ph)NHBOC, n =1].

DiPEA (71.6 µL, 0.40 mmol) was added to a solution of compound (2*S**,7a*R**)-I (wherein X = OMe, R₁ = R₂ = R₃ = R₄ = H, n =1; prepared according to the example 10) (0.041g, 0.20 mmol), L-Boc-NH-Phe-OH (0.054 g, 0.20 mmol) and PyBroP (0.096 g, 0.20 mmol) in CH₂Cl₂ (2 mL) with cooling in an ice/water bath. The reaction mixture was treated following the same procedure described in the example 9. The crude product was purified by column chromatography on silica gel (eluent: AcOEt: petroleum ether, 1.5 : 1, *R*_{*f*} = 0.20) to afford a mixture of the two diastereoisomers (2*S**,7a*R**)-I (wherein X = OMe, R₁ = R₂ = R₃ = H, R₄ = C(O)CH(CH₂Ph)NHBOC, n =1) (0.085 g, 95%). The diastereomeric mixture was partially separated by column chromatography (eluent: AcOEt: petroleum ether, 1 : 1, *R*_{*f*} = 0.18) to afford one of the two diastereoisomer (2*S**,7a*R**)-I (wherein X = OMe, R₁ = R₂ = R₃ = H, R₄ = C(O)CH(CH₂Ph)NHBOC, n =1) (0.021 g, 25%) as a white solid.

*R*_{*f*} = 0.18 (AcOEt: petroleum ether, 1 : 1); m.p = 141-144 °C; ¹H-NMR (300 MHz, CDCl₃) δ 7.33-7.19 (m, 5H), 6.40 (d, *J =* 3.9 Hz, 1H), 4.91 (br s, 1H), 4.60 (m, 1H), 4.35 (q, *J* = 6.8 Hz, 1H), 3.77 (s, 3H), 3.69 (dt, *J* = 11.3, 7.8, Hz, 1H), 3.21 (dd, *J* = 12.7, 7.8 Hz, 1H), 3.15 (dd, *J* = 13.2, 6.8 Hz, 1H), 3.07 (d, *J* = 6.6 Hz, 2H), 2.48 (ddd, *J =* 12.0, 6.4, 5.4 Hz, 1H), 2.11-2.00 (m, 2H), 1.84 (dd, *J =* 12.5, 11.5 Hz, 1 H), 1.69 (dt, *J* = 12.7, 9.0 Hz, 1H), 1.41 (s, 9H).

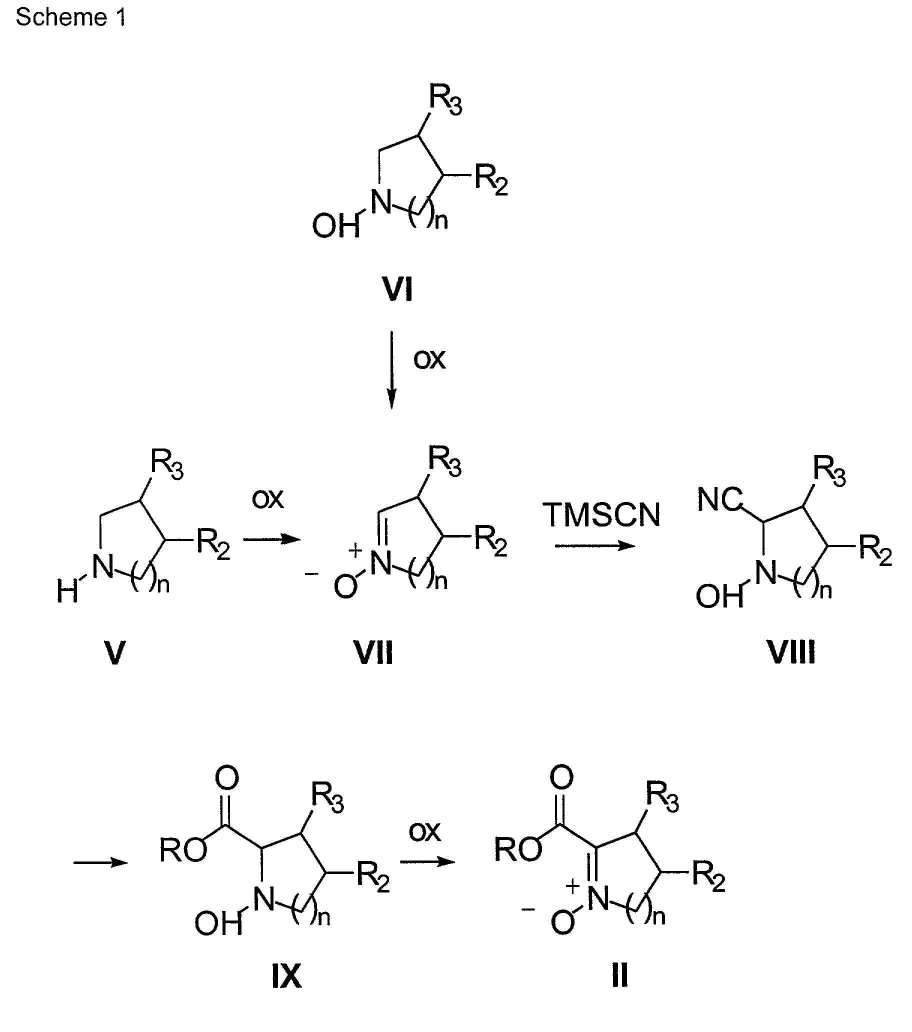

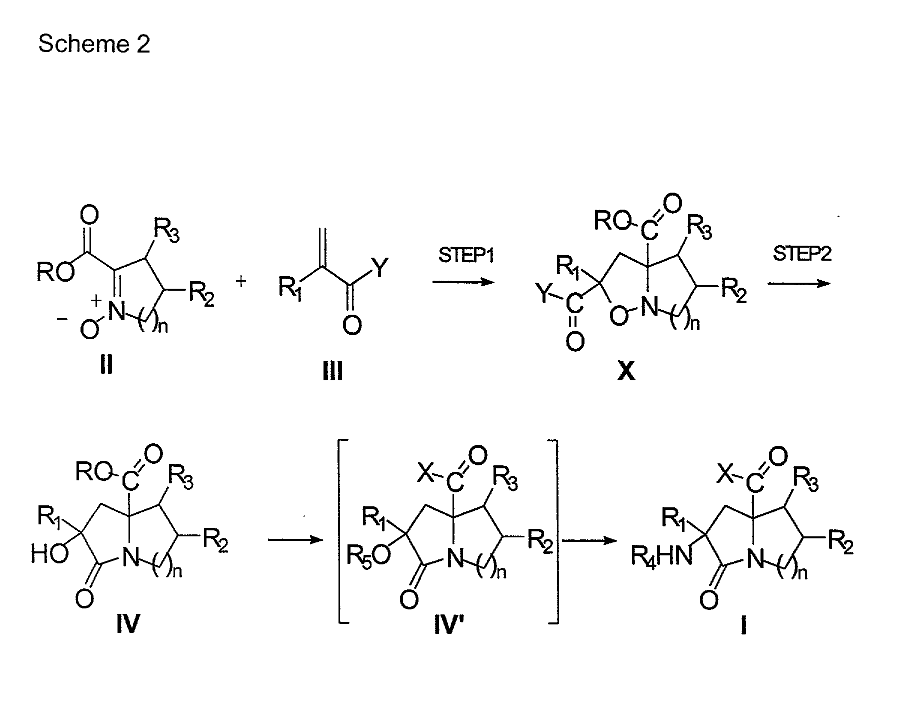

## Claims

1. Bicyclic dipeptides isosteres of general formula (I) 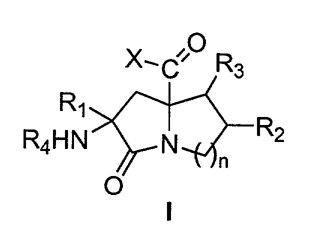 wherein:
n is an integer from 1 to 10
R₁, is chosen in the group consisting of H, C₁₋₁₆alkyl, aryl, an amino acid side-chain;
R₂, is chosen in the group consisting of H, C₁₋₁₆alkyl, OR^{I};
R₃, is chosen in the group consisting of H, C₁₋₁₆alkyl, OR^{I} or
R₂ and R₃ taken together are an aryl-fused group;
R₄, is chosen in the group consisting of H, C₁₋₁₆alkyl; a protecting group, -C(O)CH(amino acid side-chain)NR^{II}R^{III}, -C(O)(1-R^{II}-pyrrolidin-2-yl); -C(O)(1-R^{II}-piperidin-2-yl);-C(O)CH₂CH₂NR^{II}R^{III};
X is chosen in the group consisting of OR^{I}, NR^{IV}R^{V};
R^{I} is chosen in the group consisting of H, C₁₋₁₆alkyl, a protecting group;
R^{II} and R^{III}, same or different, are chosen in the group consisting of: H, C₁₋₁₆alkyl, a protecting group, -C(O)CH(amino acid side-chain)NR^{II}R^{III}, -C(O)(1-R^{II}-pyrrolidin-2-yl);-C(O)(1-R^{II}-piperidin-2-yl);-C(O)CH₂CH₂NR^{II}R^{III};
R^{IV} and R^{V}, same or different, are chosen in the group consisting of: H, C₁₋₁₆alkyl, a protecting group, -CH(amino acid side-chain)COX, -CH₂CH₂C(O)X or
R^{IV} and R^{V} taken together are: -CH(COX)CH₂CH₂CH₂-, -CH(COX)CH₂CH₂CH₂CH₂-;
the above said alkyl- and aryl-groups, being, possibly, substituted.

2. Bicyclic dipeptides isosteres of general formula (I) according to claim 1 wherein:
the protecting group is chosen in the group consisting of: methyl, ethyl, tert-butyl, benzyl, allyl esters (protecting the group COOH); Boc, Fmoc, Cbz, Bn, Bz (protecting the group NH); benzyl, tert-butyl; acetals, esters, trialkylsilylethers (protecting the group OH);
the amino acid side-chain is chosen in the group consisting of: -H, -CH₃, -CH(CH₃)₂, -CH₂CH(CH₃)₂, -CH(CH₃)CH₂CH₃, -CH₂OH, -CH(OH)CH₃, -CH₂SH, -CH₂CH₂SCH₃, -CH₂-(phenyl), -CH₂-(phenyl)-OH, -CH₂-(1H-indol-3-yl), -CH₂COOH, -CH₂C(O)(NH₂), -CH₂CH₂COOH, -CH₂CH₂C(O)NH₂, -CH₂CH₂CH₂N(H)C(NH₂)NH, -CH₂-(1H-imidazol-4-yl), -CH₂(CH₂)₃NH₂, -CH₂(CH₂)₂CH₃, -CH₂CH₂CH₃, -phenyl, such chains being possibly protected by the usual protecting groups;
the group C₁₋₁₆ alkyl is a linear or branched alkyl radicals chosen in the group consisting of: methyl, ethyl, propyl, isopropyl, butyl, pentyl, hexyl, heptyl, octyl and so on up to hexadecyl and their corresponding isomers;
the group aryl means: phenyl, naphthyl, pyridyl, and quinolinyl;
aryl-fused group means: phenyl, naphthyl, pyridyl, and quinolinyl groups;
the above said alkyl or aryl groups being possibly substituted by one or two moieties chosen in the group consisting of: C₁₋₁₆alkyl, X, NR^{II}R^{III}, -C(O)C₁₋₁₆alkyl, -C(O)X, -CH₂X, -CH₂NR^{II}R^{III}, halogen, cyano, nitro wherein X, R^{II}, R^{III} are as above defined.

3. Compounds of formula (I) according to claim 1 represented by the following formulae:
a) (2*R**,7a*R**)-2-amino-tetrahydro-3-oxo-1*H*-pyrrolizine-7a(5*H*)-carboxylic acid allyl ester;
b) (2*S**,7a*R**)-2-amino-tetrahydro-3-oxo-1*H*-pyrrolizine-7a(5H)-carboxylic acid methyl ester;
c) (2*S**,7a*R**)-2-[[(2S)-2-[[(1,1-dimethylethoxy)carbonyl]amino]-1-oxo-3-phenylpropyl]amino]-tetrahydro-3-oxo-1*H*-pyrrolizine-7a(5*H*)-carboxylic acid methyl ester;
d) (2*R**,7a*R**)-2-[[(2S)-2-[(1,1-dimethylethoxy)carbonyl]-2-[[(9*H*-fluoren-9-ylmethoxy)carbonyl]amino]-1-oxopropyl]amino]-tetrahydro-3-oxo-1*H*-pyrrolizine-7a(5*H*)-carboxylic acid allyl ester.

4. Process for the preparation of compounds of formula (I) according to claim 1 wherein:
compounds of general formula (II) and (III) 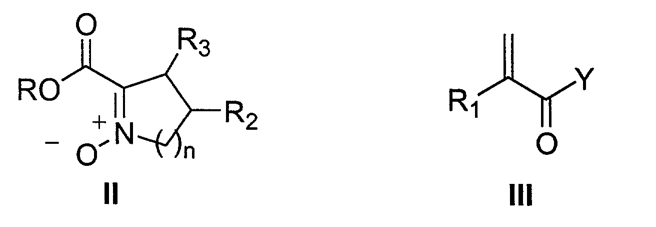 wherein:
n, R₁, R₂ and R₃ are as above defined;
R is H, or a suitable protecting group as above defined, or a chiral auxiliary;
Y is chosen in the group consisting of OR^{VI}, NR^{VII}R^{VIII};
R^{VI}, is chosen in the group consisting of H, C₁₋₄alkyl, a protecting group, a chiral auxiliary;
R^{VII} and R^{VIII}; same or different, are chosen in the group consisting of: H, C₁₋₄alkyl, a protecting group, a chiral auxiliary
are cycloadded to give the isoxazolidine derivative (X) 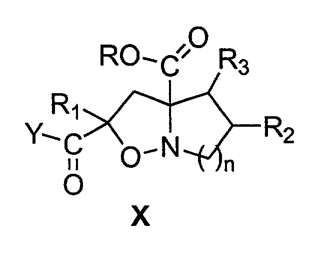 which is reductively opened and then intramoleculary cyclized to give the bicyclic lactam intermediate (IV) 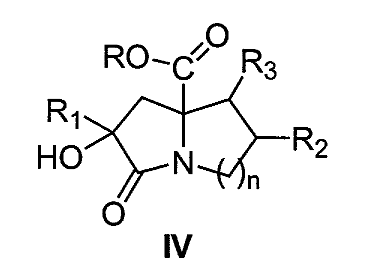 which is finally transformed into the compound of formula (I) by conversion of the hydroxy function in amine through any conventional method.

5. Process according to claim 4 wherein the reaction between compounds (II) and (III) is performed in water at a temperature comprised between 25 °C and 100 °C for a time comprised between 1 and 24 hours.

6. Process according to claim 4 wherein the reduction of the N-O bond of compound (X) is performed using Pd(OH)₂ on carbon in methanol at 1 atm and room temperature for a time comprised between 1 and 12 hours in the presence of 10 equivalents of CH₃CO₂H.

7. Process according to Claim 4 wherein, once the product of formula (VI) has been obtained, its OR group is substituted with an X moiety wherein X is as previously defined.

8. Compound of formula (X) 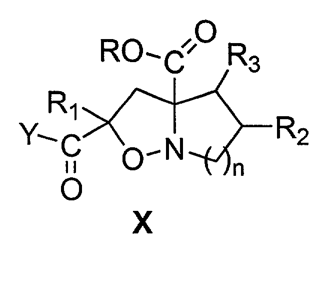 wherein: n, R, R_{1,}R₂, R₃ and Y are as above defined

9. Compound of formula (IV) 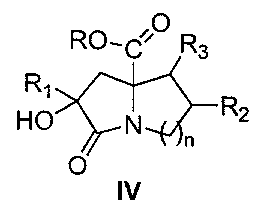 wherein: n, R, R_{1,}R₂ and R₃ are as above defined

10. Compound of formula (XI) 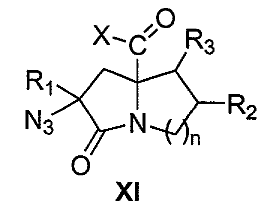 wherein: n, R_{1,},R₂,R₃ and X are as above defined

11. Use of the compounds of formula (I) according to Claim 1 for the design of peptide mimetics.

12. Use of the compounds of formula (I) according to Claim 1 for inducing beta-turn when incorporated into a peptide.
